Europäisches Patentamt

European Patent Office

Office européen des brevets

(11) Publication number: **0 228 152**
**B1**

(12) **EUROPEAN PATENT SPECIFICATION**

(45) Date of publication of patent specification: **08.08.90**

(21) Application number: **86306651.0**

(22) Date of filing: **28.08.86**

(51) Int. Cl.⁵: **A 61 M 37/00, A 61 F 7/00**

(54) Therapeutic heating apparatus.

(30) Priority: **02.10.85 KR 12843 u**

(43) Date of publication of application:
**08.07.87 Bulletin 87/28**

(45) Publication of the grant of the patent:
**08.08.90 Bulletin 90/32**

(84) Designated Contracting States:
**AT BE CH DE FR GB IT LI LU NL SE**

(56) References cited:
**US-A-4 090 517**
**US-A-4 381 009**

(73) Proprietor: **Jei, Chung Choi**
**491, Seocho-Dong Kangnam-Ku**
**Seoul (KR)**

(72) Inventor: **Jei, Chung Choi**
**491, Seocho-Dong Kangnam-Ku**
**Seoul (KR)**

(74) Representative: **Votier, Sidney David et al**
**CARPMAELS & RANSFORD 43, Bloomsbury**
**Square**
**London WC1A 2RA (GB)**

Courier Press, Leamington Spa, England.

## Description

The present invention relates to a therapeutic heating apparatus and more particularly to an electric heating cup which comprises a cotton pad containing extracts of mugwort herb or other pharmaceutical ingredients and a heating member for vaporizing the ingredients in the pad. The therapeutic heating appliance is utilized by the patient for treating various portions of the body. Thus, the vapor produced from the medical ingredients of the pad penetrate the body of the patient due to heating energy and pressure.

Many types of therapeutic heating devices, such as disclosed in US—A—4090517, are well known in the art. It is also known to treat portions of the body by placing a dry mugwort pill on the relevant portion and burning the pill. However, this treatment can result in burning the human body. Known fumigators which are used to burn mugwort do not control the temperature of the contacting surfaces. Also, it is difficult to treat the sloped portions of the body by using conventional therapeutic heating devices.

It is therefore an object of the present invention to provide a therapeutic heating apparatus which does not produce herb ash or smoking gas.

According to the present invention there is provided a therapeutic heating apparatus for the human body comprising:

a base member,
a heater assembled with the base member,
a heat conductor assembled with the heater, and
a cotton member adapted to contain a medicinal ingredient absorbed therein,
characterised in that the heat conductor is a conducting plate containing spring-biased ball means for attachment to the heater and further characterised by a cotton cover disposed to cover the cotton member and the heat conducting plate whereby, upon the heating of the heater, the medicinal ingredients are evaporated to penetrate any desired portion of the body.

Thus, the vapor produced from the medicinal ingredients of the pad by heat penetrate into the body of the patient with heating energy and pressure for treating and healing up the human body.

Specific embodiments of the present invention are now described by way of example only with reference to the accompanying drawings, in which:-

Figure 1 is an exploded view showing the basic components of the heating apparatus according to the present invention;

Figure 2 is a perspective view of the heat conducting plate containing a semicircular elastic ring;

Figure 3 is a sectional view of the heating apparatus showing a preferred embodiment of the therapeutic heating device according to the present invention;

Figure 4 is a sectional view of the heat conducting plate with a concave configuration according to the present invention; and

Figure 5 is a sectional view of the heat conducting plate with a convex configuration according to the present invention.

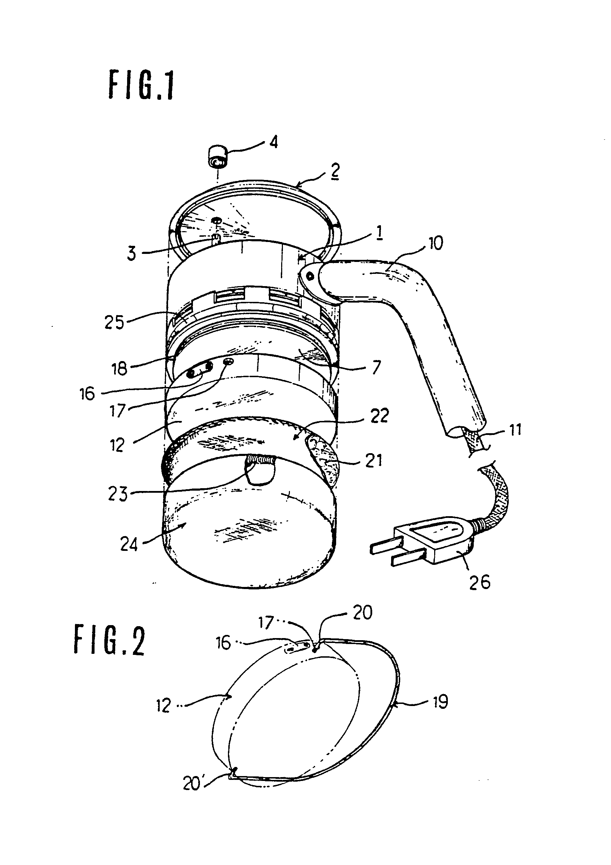

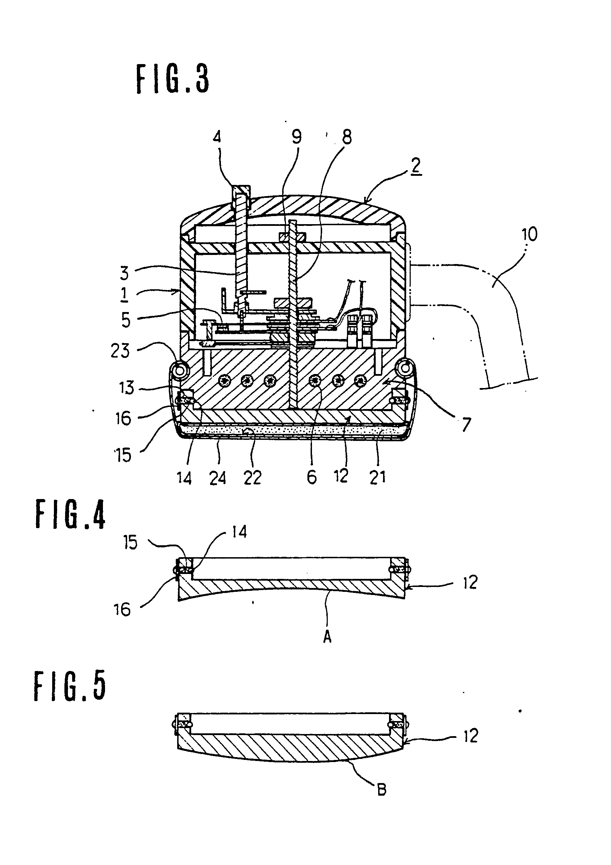

Referring now in detail to the drawings for the purpose of illustrating the present invention, the therapeutic heating apparatus as shown in Fig. 1 comprises a base member 1 containing a handle 10, a cover 2 for covering the base member, an electrical heater 7 attached to the base by a bolt 8 and a nut 9 (see Fig. 3), a heat conducting plate 12 attached to the heater 7, a dry cotton pad 22 adapted to contain mugwort herb (Artemisia Vulgaris) tonic and/or other medicinal ingredients 21 and a cotton cover 24 which covers the conducting plate 12 and the cotton pad 22. The base member 1 contains a thermostat 5 which connects with the vertical element 3 which passes through the upper portion of the base member 1 and extends through the cover 2 as shown in Fig. 3. A temperature control nub 4 is utilized to set the thermostat 5 as well as to close the cover 2 on the base member 1. The electrical heater includes a series of heating wires 6 which connect to a cord 11 which extends through the handle 10. The cord 11 contains a plug 26. The handle 10 is made of an elastic material such as a soft plastic so that it can be slidably turned to several directions. Also, the length of the handle 10 can be adjusted for easy handling.

The heat conducting plate 12 can be formed as a concave surface A (Fig. 4) or a convex surface B (Fig. 5) to accommodate the curvatures of the affected portions of the human body. Fig. 2 shows the heat conducting plate 12 which includes apertures 17 symmetrically disposed for holding a semicircular elastic ring 19, having curvatures 20, 20′ at both ends thereof, whereby the user can separate the plate 12 from the heater 7. Also, balls 14 are provided with springs 15 and extend through holes 13 disposed in the walls of the conducting plate 12 for spring biased engagement with grooves 18 disposed in the lower portion of the heater 7. A small plate 16 facilitates the attachment of the spring 15 to the conducting plate on both sides. The pad 22 contains a plurality of cotton pills containing mugwort herb tonic or medicinal ingredients absorbed therein and in a completely dried state. The cotton cover 24 for covering the conducting plate and the pad, has a coil spring 23 disposed at its circumferential end whereby the coil spring can attach to a ring groove 25 provided in the heater.

When it is desired to treat an affected portion of the human body, the user should first select the most appropriate heat conducting plate. For example, for the legs or arms, it is better for the user to select a plate formed with a concave surface as shown in Fig. 4 and ultimately, for the breast, it may be desirable to select a convex surface as shown in Fig. 5. Also, the user can select the medicinal ingredients according to the kind of disease. To assemble the device the heater is connected to the base member. The heat conducting plate is attached to the heater and the pad and cotton cover are attached to the heater by the ring spring 23. Prior to assembly, the pad is pre-wetted.

When the plug 26 is connected to a power source, the pre-wetted pad is heated by the electric heater and the heat conducting plate. The vapor evaporating from the pad can then be used to penetrate any desired portion of the human body. The heat energy and pressure simultaneously act to treat the effected portion. Also, the temperature can be controlled by using the thermostat nub 4 on the cover 2. The cotton cover 24 fully covers the pad and the heat conducting plate so that the user cannot be burned by the device. The apparatus of the present invention cannot adversely effect the environment since there is no burning system.

## Claims

1. A therapeutic heating apparatus for the human body comprising:
a base member (1),
a heater (7) assembled with the base member (1),
a heat conductor assembled with the heater, and
a cotton member (22) adapted to contain a medicinal ingredient (21) absorbed therein,
characterised in that the heat conductor is a conducting plate (12) containing spring-biased ball means (14, 15) for attachment to the heater (7) and further characterised by a cotton cover (24) disposed to cover the cotton member (22) and the heat conducting plate (12) whereby, upon the heating of the heater (7), the medicinal ingredients (21) are evaporated to penetrate any desired portion of the body.

2. The therapeutic heating apparatus of claim 1 wherein the base member (1) includes a thermostat (5) connected to the electrical heater (7) for controlling the heating temperature.

3. The therapeutic heating apparatus of claim 1 or claim 2 wherein the base member (1) is provided with a handle (10) which houses an electric cord (11).

4. The therapeutic heating apparatus of any preceding claim wherein the heat conducting plate (12) includes means (17) for attaching a semicircular elastic ring (19) thereto.

5. The therapeutic heating apparatus of claim 1 wherein the cotton member (22) comprises a plurality of cotton pills impregnated with mugwort herb or other medicinal ingredients.

6. The therapeutic heating apparatus of any preceding claim wherein the heater (7) contains a groove (18) for receiving the spring biased balls (14) and the cover (24) contains a ring spring (23) disposed at the periphery thereof for covering the cotton member (22) and the heat conducting plate (12).

7. The therapeutic heating apparatus of any preceding claim wherein the heat conducting plate (12) is convex.

8. The therapeutic heating apparatus of any one of claims 1 to 6 wherein the heat conducting plate (12) is concave.

## Patentansprüche

1. Therapeutische Heizvorrichtung für den menschlichen Körper mit
einem Basisglied (1)
einer Heizeinrichtung (7), die mit dem Basisglied (1) zusammengesetzt ist,
einer Wärmeleiteinrichtung, die mit der Heizeinrichtung zusammengesetzt ist, und
einem Baumwollglied (22), welches ein darin absorbiertes medizinisches Ingredienz (21) enthalten kann,
dadurch gekennzeichnet, daß die Wärmeleiteinrichtung eine leitende Platte (12) ist, die eine federvorgespannte Kugeleinrichtung (14, 15) zur Anbringung an der Heizeinrichtung (7) enthält und weiter gekennzeichnet durch eine Baumwollabdeckung (24), die zur Abdeckung des Baumwollgliedes (22) und der Wärmeleitplatte (12) angeordnet ist, wodurch bei dem Erwärmen bzw. Erhitzen der Heizeinrichtung (7) die medizinischen Ingredienzien (21) verdampft werden, um in einem gewünschten Teil des Körpers einzudringen.

2. Therapeutische Heizvorrichtung nach Anspruch 1, bei welcher das Basisglied (1) einen Thermostaten (5) umfaßt, der mit der elektrischen Heizeinrichtung (7) zur Steuerung der Heiztemperatur verbunden ist.

3. Therapeutische Heizvorrichtung nach Anspruch 1 oder 2, in welcher das Basisglied (1) mit einem Handgriff (10) vorgesehen ist, in welchem eine elektrische Leitung (11) untergebracht ist.

4. Therapeutische Heizvorrichtung nach einem der vorhergehenden Ansprüche, in welcher die Wärmeleitplatte (12) Mittel (17) zur Anbringung eines halbkreisförmigen elastischen Ringes (19) daran umfaßt.

5. Therapeutische Heizvorrichtung nach Anspruch 1, in welcher das Baumwollglied (22) eine Vielzahl von Baumwollpills bzw. Baumwollfaserkügelchen umfaßt, die mit Beifußkräutern oder anderen medizinischen Ingredienzien imprägniert sind.

6. Therapeutische Heizvorrichtung nach einem der vorhergehenden Ansprüche, bei welcher die Heizeinrichtung (7) eine Nut (18) zur Aufnahme der federvorgespannten Kugeln (14) aufweist und die Abdeckung (24) eine Ringfeder (23) umfaßt, die an deren Umfang zur Abdeckung des Baumwollgliedes (22) und Wärmeleitungsplatte (12) umfaßt.

7. Therapeutische Heizvorrichtung nach einem der vorhergehenden Ansprüche, bei welcher die Wärmeleitplatte (12) konvex ist.

8. Therapeutische Heizvorrichtung nach einem der Ansprüche 1 bis 6, bei welcher die Wärmeleitplatte (12) konkav ist.

## Revendications

1. Un dispositif de chauffage thérapeutique pour le corps humain comprenant:
un élément de base (1),

un élément chauffant (7) assemblé avec l'élément de base (1),

un conducteur thermique assemblé avec l'élément chauffant, et

un élément en coton (22) adapté pour contenir un ingrédient médicinal (21) absorbé à l'intérieur, caractérisé en ce que le conducteur thermique est une plaque conductrice (12) contenant des moyens en forme de bille (14, 15) soumises à l'action d'un ressort, pour fixation sur l'élément chauffant (7), et caractérisé en outre par un capuchon de coton (24) agencé pour recouvrir l'élément de coton (22) et la plaque thermoconductrice (12) de telle sorte que lors du chauffage par l'élément chauffant (7), les ingrédients médicinaux (21) soient évaporés, de manière à pénétrer dans une partie quelconque désirée du corps.

2. Le dispositif de chauffage thérapeutique selon la revendication 1, dans lequel l'élément de base (1) comprend un thermostat (5) relié à l'élément chauffant électrique (7) pour le réglage de la température de chauffage.

3. Le dispositif de chauffage thérapeutique selon la revendication 1 ou 2, dans lequel l'élément de base (1) est pourvu d'une poignée (10) logeant un cordon électrique (11).

4. Le dispositif de chauffage thérapeutique selon l'une quelconque des précédentes revendications, dans lequel la plaque thermoconductrice (12) comprend des moyens (17) pour fixer une bague élastique semi-circulaire (19) sur celle-ci.

5. Le dispositif de chauffage thérapeutique selon la revendication 1, dans lequel l'élément de coton (22) comprend une pluralité de boulettes de coton imprégnées d'armoise commune ou d'autres ingrédients médicinaux.

6. Le dispositif de chauffage thérapeutique selon l'une quelconque des précédentes revendications, dans lequel l'élément chauffant (7) contient une rainure (18) pour recevoir des billes (14) soumises à l'action d'un ressort et le capuchon (24) contient un ressort annulaire (23) disposé sur la périphérie de celui-ci, pour recouvrir l'élément de coton (22) et la plaque thermoconductrice (12).

7. Le dispositif de chauffage thérapeutique selon l'une quelconque des précédentes revendications, dans lequel la plaque thermoconductrice est convexe.

8. Le dispositif de chauffage thérapeutique selon l'une quelconque des revendications 1 à 6, dans lequel la plaque thermoconductrice (12) est concave.

# FIG.1

# FIG.2

# FIG.3

# FIG.4

# FIG.5